# EUROPEAN PATENT SPECIFICATION

(11) **EP 4 037 536 B1**
(45) Date of publication and mention of the grant of the patent: **26.06.2024**
(21) Application number: 20776194.1
(22) Date of filing: 29.09.2020
(51) Int. Cl.: A61B 1/00, A61B 1/005

(54) **ENDOSCOPY DEVICE WITH ORIENTABLE HEAD**
ENDOSKOPIEVORRICHTUNG MIT AUSRICHTBAREM KOPF
DISPOSITIF D'ENDOSCOPIE À TÊTE ORIENTABLE

(30) Priority: 03.10.2019 IT 201900017807
(43) Date of publication of application: 10.08.2022
(73) Proprietor: Bosi, Carlo Alberto, 41037 Mirandola (IT)
(72) Inventor: FERMI, Enrico, 29122 Piacenza (IT); BOSI, Carlo Alberto, 41037 Mirandola (IT)
(74) Representative: Modiano, Micaela Nadia
(86) International application number: PCT/EP2020/077265
(87) International publication number: WO 2021/063966

(56) References cited:
- WO-A1-2013/179586
- JP-A- 2002 112 956
- US-A- 4 825 850
- US-A1- 2012 100 729

## Description

The present invention relates to an endoscopy device with an orientable head.

As is known, in the medical sector, endoscopy is a method of exploration that makes it possible to view the inside of an anatomical cavity by using adapted biomedical devices, called endoscopes, which are widely employed to carry out diagnostic examinations and/or healthcare interventions.

In particular, flexible endoscopes with an orientable head are known, which are constituted substantially by a grip, also known as a handpiece, which is connected to a flexible insertion tube which is provided at the distal end with a video camera or other optical vision system and with a light source, generally using LEDs or optic fibers. The end portion of the insertion tube proximate to the distal end can be moved using an orientation system which is provided with adjustment elements which are arranged on the handpiece and are connected to the aforesaid end by flexible actuation elements which pass through the inside of the insertion tube. The orientation system can enable the operator to orient the tip on a plane, in two mutually opposite directions, or on two mutually perpendicular planes, arranged in mutually opposite pairs in four directions. Along the insertion tube there can also be at least one auxiliary channel for the passage of fluids, in outflow or in intake, and/or of operating instruments.

Depending on the scope of application, there are different types of endoscopes such as, for example, cystoscopes, nasopharyngoscopes, gastroscopes, colonoscopes, bronchoscopes, otoscopes or other similar devices.

In order to prevent cross-contamination between patients, such devices must be carefully cleaned and sterilized after each use.

The sterilization step is critical, both because of the risks deriving from any errors, and because of the costs of this treatment and the wait times to make the instrument available again.

Alternatively, endoscopic instruments are known with an orientable tip that are fully single-use, which ensure an optimal level of hygiene but which are extremely expensive, to the point that their diffusion and possibility of use are limited. Among other things, the use of such single-use endoscopes entails the production of considerable amounts of special waste to be managed.

These conventional endoscopes with an orientable tip are not devoid of drawbacks, among which is the fact that reusable ones entail all the problems described above associated with sterilization, while single-use ones are excessively expensive.

It should be noted that in the field of endoscopes that do not allow the operator to orientate the tip of the insertion tube, solutions are known which have a reusable handpiece connected to a single-use insertion tube, but this type of solution cannot be applied to flexible endoscopes with an orientable tip because the orientation system extends both in the handpiece and along the insertion tube.

Prior art document US 2012/100729 discloses an endoscopy device according to the preamble of independent claim 1.

The aim of the present invention is to eliminate the above mentioned drawbacks in the background art by providing an endoscopy device with an orientable head that makes it possible to avoid the necessity of sterilizing the insertion tube, while still eliminating any risk of cross-contamination between patients and offering a low purchase cost, such as not to hinder the diffusion and possibility of its use.

Within this aim, an object of the present invention is to provide an endoscopy device that is practical and immediate in use, in order to allow medical personnel to execute diagnostic and operating procedures using conventional methods, without complications or inconveniences for the operators.

Another object of the present invention is to provide an endoscopy device that is reliable in use, so as to ensure the safety of patients and the efficacy of the procedures carried out.

Another object of the present invention, with respect to known single-use solutions, is to provide an endoscopy device that makes it possible to reduce the amount of special waste to be managed.

Last but not least, another object of the present invention is to provide an endoscopy device that has a simple structure which is easy and practical to implement, safe in use and effective in operation, and low-cost.

This aim and these objects are achieved by an endoscopy device with an orientable head according to claim 1, optionally provided with one or more of the characteristics recited in the dependent claims.

Further characteristics and advantages of the present invention will become more apparent from the detailed description of some preferred, but not exclusive, embodiments of the invention, which are illustrated for the purposes of non-limiting example in the accompanying drawings wherein:
Figure 1 is a perspective view of a first embodiment of an endoscopy device with an orientable head, according to the invention;
Figure 1a is an enlarged-scale front view of a possible embodiment of the distal end of the insertion tube of the device of Figure 1;
Figure 2 is a partially exploded perspective view of the device of Figure 1;
Figure 3 is a schematic and partial cross-sectional view of the device in the first embodiment;
Figures 4 and 5 are partially transparent perspective views that show the operation of the orientation system according to the first embodiment;
Figure 6 is an enlarged-scale perspective view of the joint and of the connection means of the first embodiment of the device according to the invention, with the locking means in exploded view;
Figure 7 is a view like Figure 6 with the first body and second body of the joint decoupled;
Figure 8 is a view like Figure 7, in further exploded view;
Figure 9 is a front elevation view of the joint of the device according to the invention in the first embodiment;
Figure 10 is a cross-sectional view taken along the line X-X in Figure 9;
Figure 11 is a view like Figure 3 of a variation of the device according to the invention in the first embodiment;
Figure 11a is an enlarged-scale view of a possible embodiment of the distal end of the insertion tube of the device of Figure 1;
Figure 12 is a perspective view of a second embodiment of the device according to the invention;
Figure 13 is a perspective exploded view of the device of Figure 12.

With particular reference to the figures, the reference numeral 1 generally designates an endoscopy device with an orientable head.

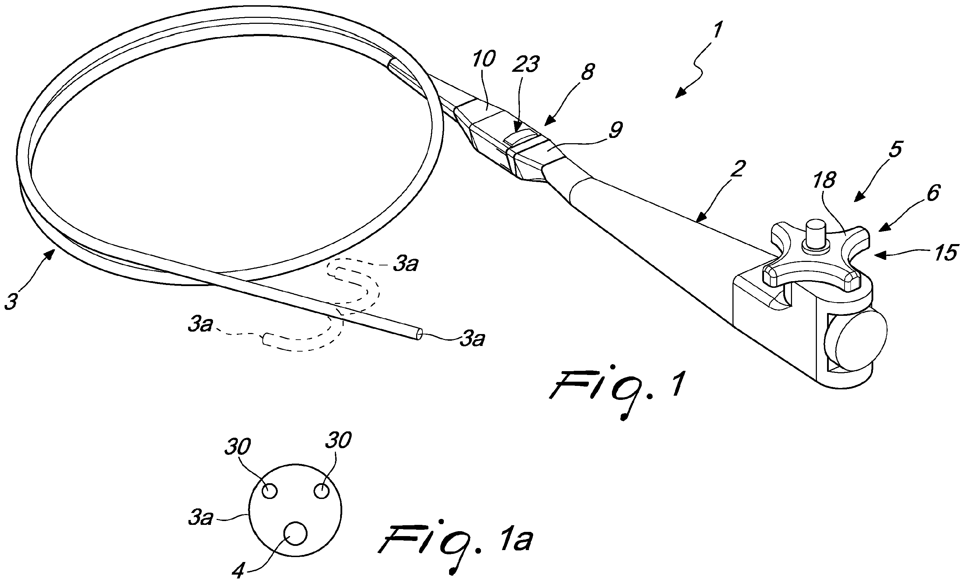

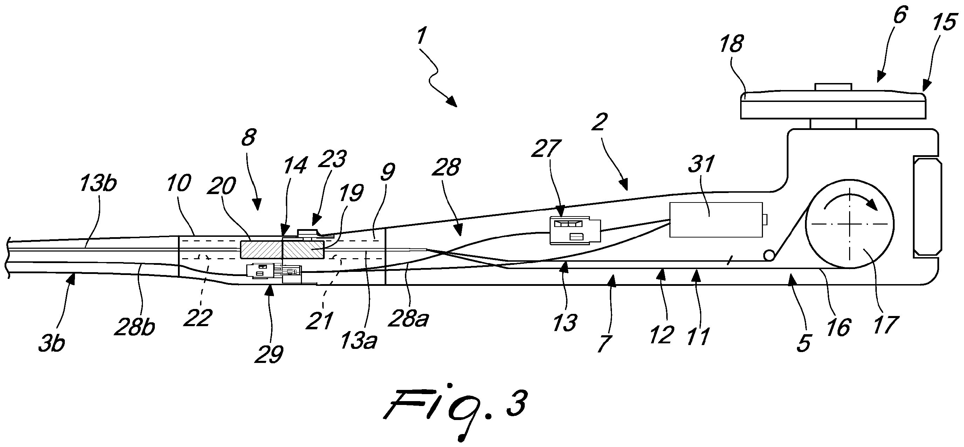

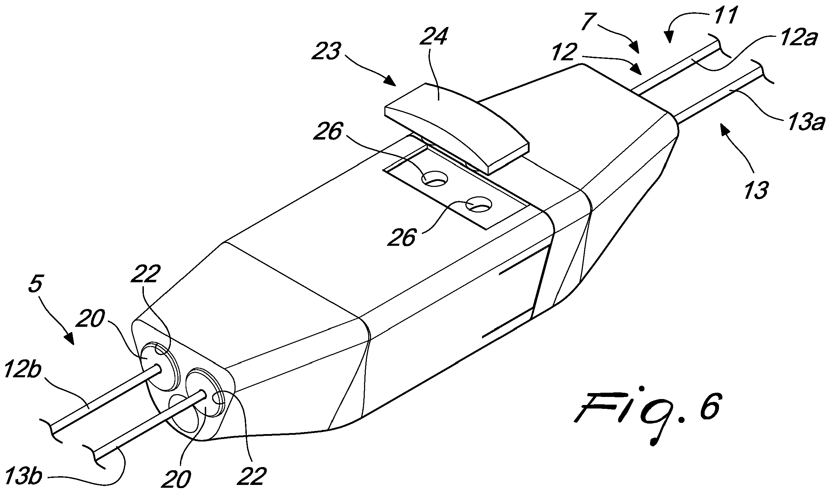

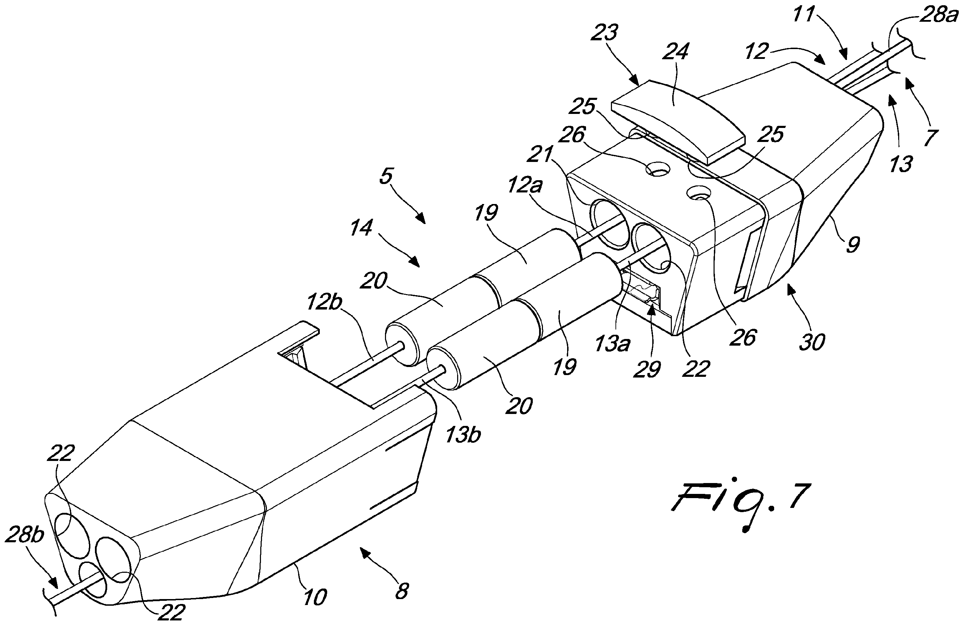

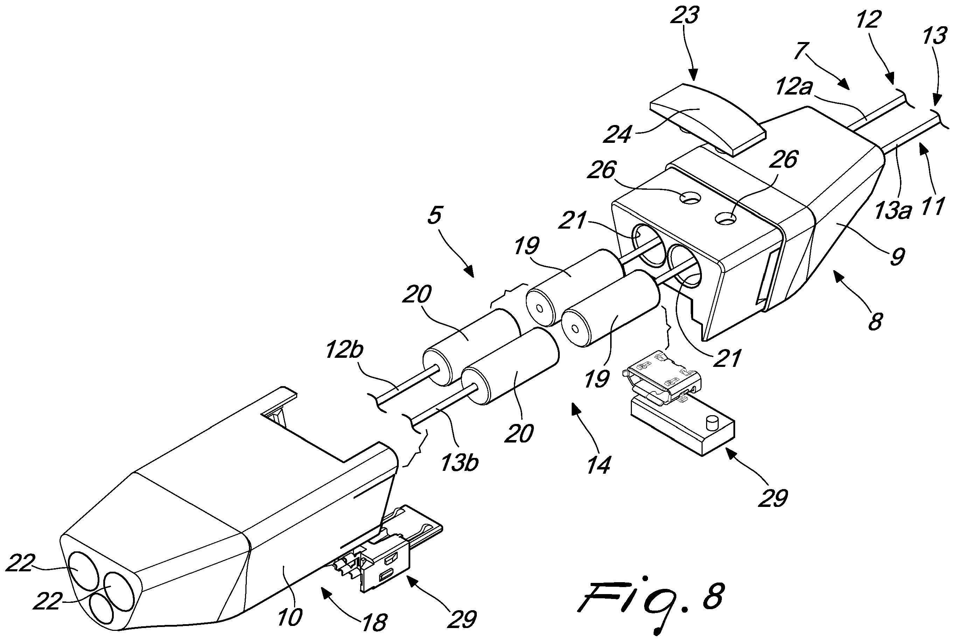

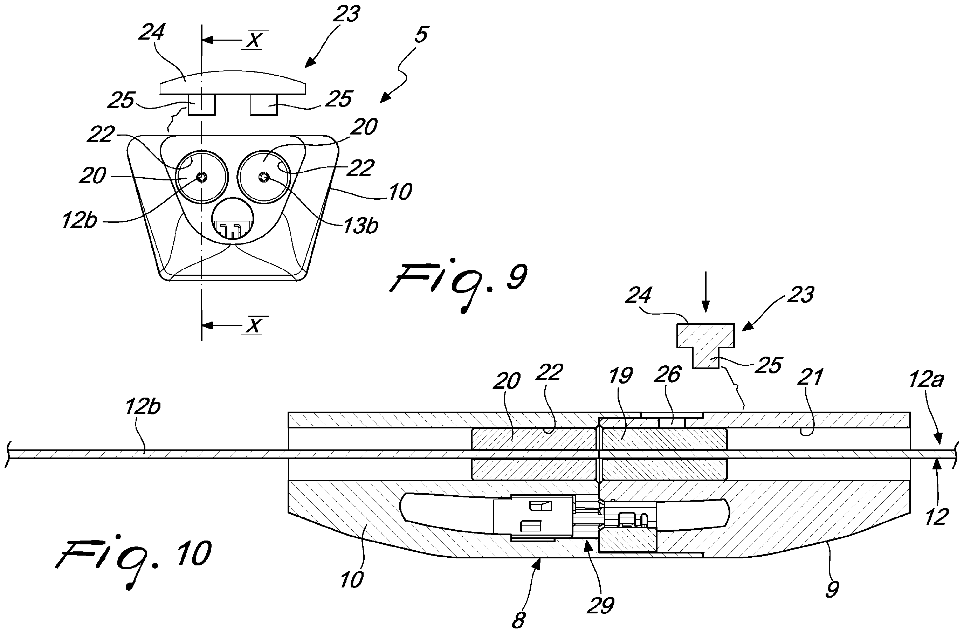

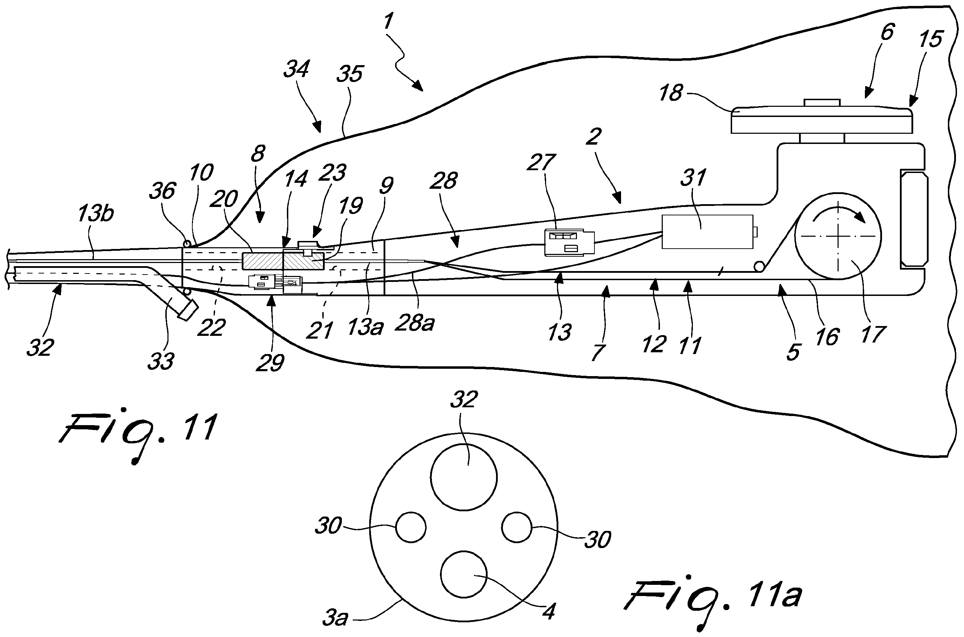

In more detail the device 1 is shown as a flexible, orientable endoscope for diagnostic use (Figures 1-10, 12-13) or for diagnostic and operating use (Figures 11 and 11 a), and the same inventive concept can be incorporated in a device for the endoscopic exploration of different anatomical cavities, such as for example the bladder, the colon, the uterus, or the stomach, simply by varying the dimensions and the shape of the insertion tube 3 according to methods that are known to the person skilled in the art.

The device 1 comprises grip means 2, called a "handpiece", for gripping and movement by an operator during use, which are associated with a flexible insertion tube 3 which is provided at the distal end 3a with optical vision means 4. The device 1, furthermore, is provided with an orientation system 5 for the end portion of the insertion tube 3 proximate to the distal end 3a, which comprises adjustment means 6 which can be actuated by an operator and are associated with the grip means 2, and flexible actuation means 7 which cooperate with the adjustment means 6 and are arranged so as to pass through the insertion tube 3 and are associated with the distal end 3a of the tube.

The term "distal end" means the end that, during use, is further from the operator who is using the device 1 by gripping the grip means 2; the term "proximal end", on the contrary, means the end that is nearer to the operator.

The grip means 2 are preferably internally hollow, so as to accommodate inside them some components which will be explained in detail in the description below.

The optical vision means 4 can be constituted, for example, by a video camera of the "chip-on-tip" type, accommodated inside the insertion tube 3 at the corresponding distal end 3a and paired with an optical lens positioned on that end, not explained in detail.

It should be noted that the distal end 3a and the associated optical lens can be variously contoured depending on the requirements of the specific application of the device 1.

The shape, the dimensions and the materials with which it is made can vary on the basis of the intended use of the device 1.

The device 1 comprises a joint 8 interposed between the grip means 2 and the insertion tube 3, which comprises a first body 9, stably associated with the grip means 2, and a second body 10, stably associated with the insertion tube 3, which can be associated temporarily with each other.

The expression "stably associated" means integrally coupled; the expression "can be associated temporarily" means that they can be coupled and uncoupled repeatedly.

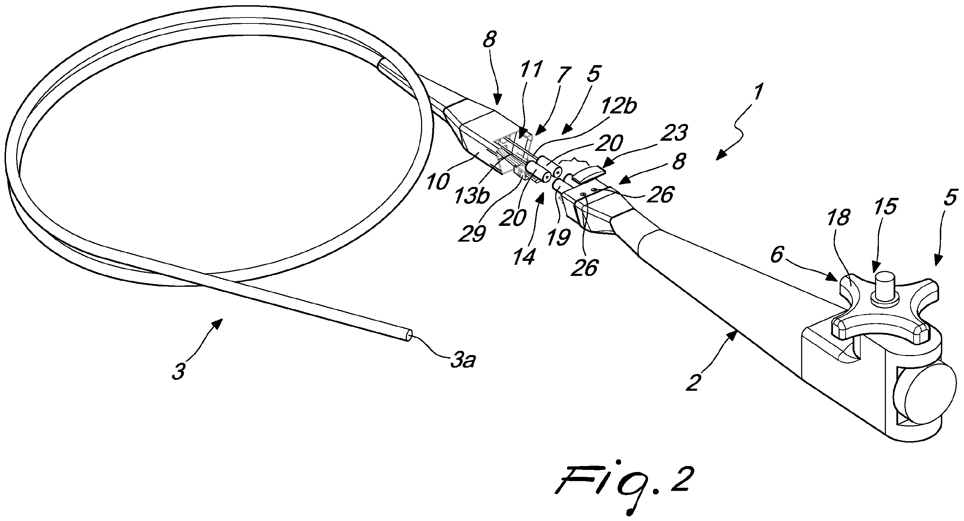

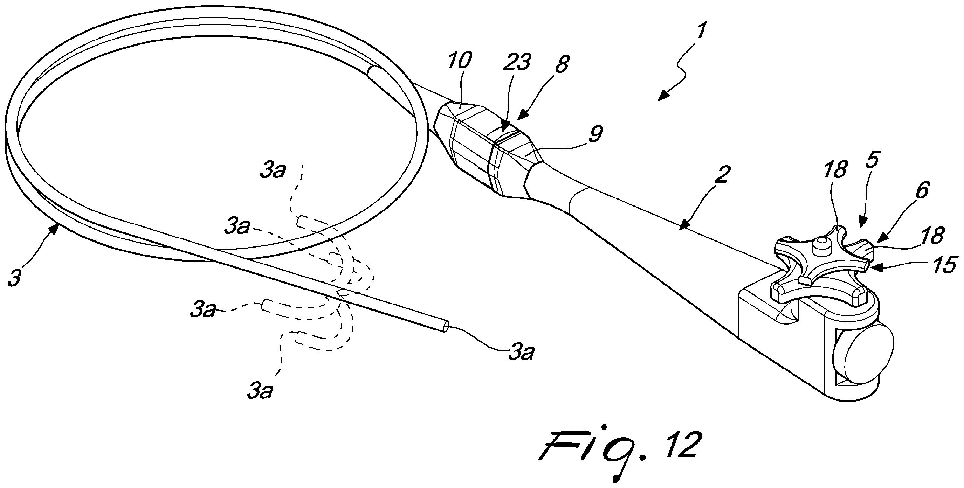

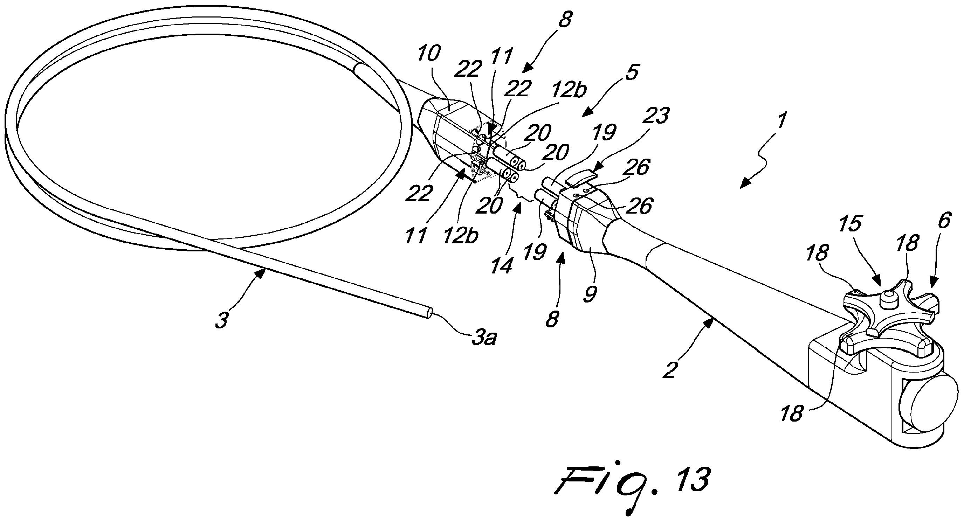

The first body and the second body 9 and 10 are therefore adapted to assume, alternatively, a coupled configuration (Figures 1 and 12), in which they are temporarily connected to each other by way of reversible interlocking elements or the like, and a decoupled configuration (Figures 2 and 13), in which they are disengaged from each other.

In the preferred embodiments shown, the first body 9 is associated with the grip means 2 and the second body 10 with the proximal end 3b of the insertion tube 3.

The possibility is not ruled out, however, that in an alternative embodiment the grip means 2 can have a tube joining segment connected to the insertion tube 3 by way of the joint 8.

According to the invention the flexible actuation means 7 comprise at least one pair 11 of flexible elements 12 and 13, each one of which is sectioned into a first portion 12a or 13a which is associated with the adjustment means 6 and is inserted so as to pass through the grip means 2 and the first body 9, and into a second portion 12b or 13b which is associated with the distal end 3a and is inserted so as to pass through the insertion tube 3 and the second body 10. Furthermore there are temporary connection means 14 which are interposed between the first portion and the second portion, 12a and 12b or 13a and 13b, of each one of the flexible elements 12 or 13.

Such flexible elements 12 or 13 are constituted by drawstrings of metallic cable or the like, according to solutions that are known to the person skilled in the art.

According to the invention, by decoupling the first body and the second body 9 and 10 and the temporary connection means 14 of each flexible element 12 or 13, it is possible to separate the grip means 2 and the insertion tube 3. In this manner it is possible to provide reusable grip means 2 and a single-use insertion tube 3, to be replaced at each use.

The invention makes it possible, therefore, to eliminate the necessity of sterilizing the insertion tube after use, without risks of contamination for patients and considerably reducing the costs of the device 1 with respect to conventional fully single-use devices, since the grip means 2, with the corresponding components associated with them, can be reused without being sterilized because they are not contaminated while carrying out the medical procedures.

The adjustment means 6 comprise at least one adjustment element 15 which is supported so that it can rotate by the grip means 2. Furthermore, the flexible elements 12 and 13 of the at least one pair 11 are mutually connected by an intermediate portion 16 which is wrapped around the aforementioned adjustment element 15 and has a portion that is integral therewith.

Actuating the rotation of the adjustment element 15 in one direction or in the other therefore activates the winding thereon of one of the flexible elements 12 or 13 and the unwinding of the other element, causing the flexion of the end portion of the insertion tube 3 in one direction or in the other.

In this manner the flexible elements 12 and 13 of the at least one pair 11 and the intermediate portion 16 form a single body.

Alternatively, the flexible elements 12 and 13 of the at least one pair 11 could be constituted by respective portions each of which has one end fixed to the adjustment element 15 and the opposite end connected to the distal end 3a.

The adjustment element 15 is constituted by a pulley 17, or the like, which is supported so that it can rotate inside the grip means 2 and is connected to an actuation knob 18 that can be accessed from outside, such elements non being described and shown in detail as they are known to the person skilled in the art.

The profile of the pulley 17 could be contoured according to the shape structure of the flexible elements 12 and 13.

In the preferred embodiment, the temporary connection means 14 are of the magnetic type.

Alternatively, the possibility is not ruled out that such connection means could be of the mechanical type and have, between the first portion and the second portion, 12a and 12b or 13a and 13b, of each flexible element 12 or 13 of the at least one pair 11, for example, a bayonet coupling, a screw coupling, a shape-based coupling or the like.

In the preferred embodiment, the temporary connection means 14 of the magnetic type comprise, for each flexible element 12 or 13 of the at least one pair 11, a first magnet and a second magnet 19 and 20 which are associated with the free ends, respectively, of the first portion and of the second portion 12a and 12b or 13a and 13b of the flexible element and are adapted to attract each other.

For the at least one pair 11 of flexible elements 12 and 13 there are, therefore, two first magnets 19 and two second magnets 20.

Preferably the first and second magnets 19 and 20 comprise neodymium. This material, in fact, makes it possible to exert a rather intense magnetic force, even with reduced contact surfaces between the magnets 19 and 20. In more detail, the first and second magnets 19 and 20 can be constituted of an alloy of boron, iron and neodymium and be coated with an anti-oxidant layer of nickel, zinc and epoxy resin. The first and second magnets 19 and 20 are adapted to generate a magnetic field characterized by a magnetic flux density that exceeds 1,000 Gauss, and they have a coercive force that exceeds 9,800 Oe.

The first magnet and the second magnet 19 and 20 associated with each flexible element 12 and 13 of the at least one pair 11 have a substantially cylindrical shape and are accommodated so as to slide along a first channel and a second channel 21 and 22 which are mutually aligned and are provided, respectively, in the first body and in the second body 9 and 10.

For the at least one pair 11 of flexible elements 12 and 13 there are, therefore, two first channels 21 and two second channels 22 for the respective first and second magnets 19 and 20.

Furthermore, there are locking means 23 for locking the sliding of the first magnets 19 along the respective first channels 21, which are associated with the first body 9 in order to allow the decoupling by traction and/or flexing of each first magnet 19 from the respective second magnet 20.

For example the locking means 23 can take the form of at least one insert 24 provided with a pair of pins 25 which can be inserted into corresponding holes 26 provided on the first body 9 at the first channels 21. In this manner, by pressing on the insert 24, the corresponding pins 25 interfere with the first magnets 19 arranged along the first channels 21, obstructing their sliding. In this condition, after having uncoupled the second body 10 from the first body 9, it is possible to exert a traction and/or a flexion on the second magnets 20, detaching them from the first magnets.

Furthermore, the device 1 has remote data transmission means 27 for sending the signal that originates from the optical vision means 4 to a separate display system, not shown, of the type of a monitor, smart glasses worn by the operator, or other conventional device. The data transmission means 27 are accommodated inside the grip means 2, and a connection cable 28 is interposed between the data transmission means and the optical vision means 4, and is divided into a first portion 28a, which extends between the data transmission means 27 and the first body 9, and into a second portion 28b, which extends between the second body 10 and the optical vision means 4. Furthermore, there are connector means 29 between the first portion and the second portion 28a and 28b of the connection cable 28, which are interposed between the first body and the second body 9 and 10.

The data transmission means 27 can consist of a transmission module using wireless LAN or Bluetooth or another conventional wireless technology.

Preferably, the device 1 also comprises lighting means 30 which are associated with the distal end 3a of the insertion tube 3, for illuminating the region inspected by the optical vision means 4.

Such lighting means 30 can take the form, for example, of one or more LEDs associated with the distal end 3a. Alternatively, there could be a bundle of optic fibers passing along the insertion tube 3 and having the associated lighting terminals at the distal end 3a.

The device 1 can also be provided with electric power supply means 31 which are functionally associated with the optical vision means 4 and with the lighting means 30, if present, via the connection cable 28, and which are accommodated inside the grip means 2. Such electric power supply means 31 can take the form, for example, of a battery, optionally rechargeable, or the like.

Preferably the connection cable 28 and the connector means 29 are of the USB type or are provided using another technology that makes it possible to achieve the transmission of video signals and of electric current.

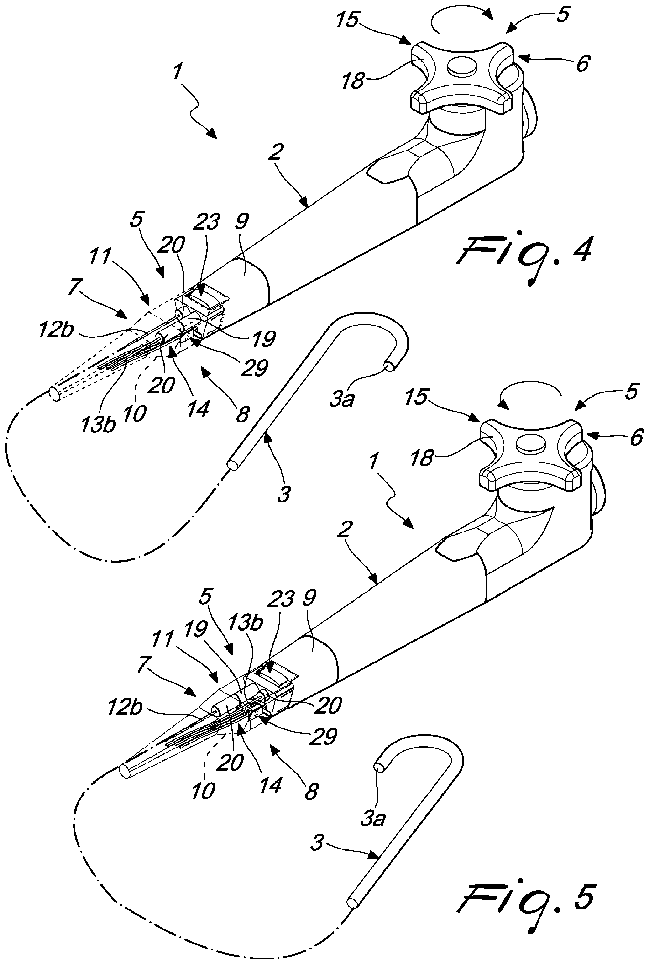

In the first embodiment of the invention, shown in Figures 1-10, the device 1 makes it possible to vary the orientation of the end portion of the insertion tube 3 in two mutually opposite directions on a single plane, as shown in Figures 4 and 5, according to the direction in which the rotation of the adjustment element 15 is actuated.

In a variation of the first embodiment of the device 1, shown in Figure 11, the insertion tube 3 comprises at least one auxiliary channel 32 which passes inside it and has a first end which is open at the distal end 3a of the tube and a second end which communicates with an access port 33 which is associated with the second body 10.

The auxiliary channel 32 can allow the passage of operating instruments or the outflow and/or the intake of fluids, gaseous (e.g. air) or liquid (e.g. sterile physiological solutions for irrigation).

In a further variation, the device 1 can have two or more auxiliary channels 32 which pass through the insertion tube 3, and these channels may also have different uses.

Optionally the device 1 can be provided with a protection element 34, shown in Figure 11: such protection element 34 is configured to protect the first body 9 of the joint, the grip means 2 and any components connected to these from any contamination, as these constitute the reusable part of the device 1.

To this end the protection element 34 comprises a tubular enclosure 35 and an enclosure retention element 36 which can be applied to the joint 8 at the second body 10, so as to isolate the entire reusable part of the device 1.

Preferably the tubular enclosure 35 is made of plastic material and is flexible; preferably it is single-use.

Preferably the retention element 36 is an elastic element, designed to engage the open inlet of the tubular enclosure 35 on the second body 10.

It should be noted that, in the presence of one or more auxiliary channels 32, the retention element 36 is positioned along the second body 10 so that the tubular enclosure 35 does not also cover the access port 33 of these channels.

Similarly, the protection element 34 can also be provided on devices 1 that do not have an auxiliary channel 32.

In a second embodiment of the device 1, shown in Figures 12 and 13, the orientation system 5 takes the form of two pairs 11 of flexible elements 12 and 13, each flexible element being divided into a first portion and a second portion, 12a and 12b or 13a and 13b, between which corresponding temporary connection means 14 are interposed.

The pairs 11 of flexible elements 12 and 13 are arranged on substantially perpendicular planes, the elements themselves being distributed angularly spaced apart by 90° in pairs, so as to allow adjustment of the orientation of the tip of the insertion tube 3 at the distal end, in four directions in mutually opposite pairs (Figure 12).

The temporary connection means 14 therefore have, for each flexible element 12 or 13, a first magnet 19 and a second magnet 20, for a total of four first magnets 19 and four second magnets 20, not all of which are visible in the figures.

The bodies 9 and 10 are provided, respectively, with four first channels 21 and with four second channels 22, in aligned and communicating pairs and distributed like the flexible elements 12 and 13, not all of which are visible in the figures.

The orientation system 5 has two adjustment elements 15 which are provided with respective knobs 18 which can be actuated independently.

The second embodiment of the device 1 can also be provided with one or more auxiliary channels 32.

In practice it has been found that the invention as described achieves the intended aim and objects and, in particular, attention is drawn to the fact that the device according to the invention makes it possible to obtain a solution that does not entail the drawbacks deriving from the necessity of sterilizing the insertion tube and which is low-cost, so as not to hinder the distribution and possibility of its use.

Furthermore, the solution according to the invention is safe for patients and it is practical and efficacious in use for operators.

Last but not least, the solution according to the invention reduces the amount of special waste to be managed, with respect to conventional fully single-use devices.

The invention, thus conceived, is susceptible of numerous modifications and variations, all of which are within the scope of the appended claims.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. An endoscopy device (1) with an orientable head, which comprises grip means (2) which are associated with a flexible insertion tube (3) which is provided at the distal end (3a) with optical vision means (4), an orientation system (5) being further provided for an end portion of said insertion tube (3) proximate to the distal end (3a), which comprises adjustment means (6) which can be actuated by an operator and are associated with said grip means (2), and flexible actuation means (7) which cooperate with said adjustment means (6) and are arranged so as to pass through said insertion tube (3) and are associated with the distal end (3a) of said tube, the endoscopy device (1) further comprising a joint (8) which is interposed between said grip means (2) and said insertion tube (3) and which comprises a first body (9) which is stably associated with said grip means (2) and a second body (10) which is stably associated with said insertion tube (3), which can be associated temporarily with each other, said flexible actuation means (7) comprising at least one pair (11) of flexible elements (12, 13), each one of which is sectioned into a first portion (12a; 13a), which is associated with said adjustment means (6) and is inserted so as to pass through said grip means (2) and said first body (9), and into a second portion (12b; 13b), which is associated with the distal end (3a) of said insertion tube (3) and is inserted so as to pass through said tube and said second body (10), and temporary connection means (14) being further provided which are interposed between the first and second portions (12a, 12b; 13a, 13b) of each one of said flexible actuation elements (12; 13), said connection means (14) being of the magnetic type and said connection means (14) comprising, for each one of said flexible actuation elements (12; 13) of said at least one pair (11), a first magnet and a second magnet (19, 20), which are associated with the free end respectively of the corresponding first portion and of the second portion (12a, 12b; 13a, 13b) and are adapted to attract each other, **characterized in that** said first magnet and said second magnet (19, 20) associated with each one of said flexible actuation elements (12; 13) of said at least one pair (11) have a substantially cylindrical shape and are accommodated so as to slide along a first channel and a second channel (21, 22) which are mutually aligned and are provided, respectively, in said first body and said second body (9, 10).

2. The device (1) according to claim 1, **characterized in that** said first and second magnets (19, 20) comprise neodymium.

3. The device (1) according to claim 1, **characterized in that** it comprises locking means (23) for locking the sliding of said first magnets (19) along the respective first channels (21), which are associated with said first body (9) in order to allow uncoupling by traction and/or flexing of the first magnet and of the second magnet (19, 20) which are associated with each one of said flexible actuation elements (12; 13) of said at least one pair (11).

4. The device (1) according to claim 1, **characterized in that** said adjustment means (6) comprise at least one adjustment element (15) which is supported so that it can rotate by said grip means (2) and **in that** the flexible elements (12, 13) of said at least one pair (11) are mutually connected by an intermediate portion (16) which is wrapped around said adjustment element (15) and has a portion that is integral therewith.

5. The device (1) according to claim 1, **characterized in that** said connection means (14) are of the reversible mechanical type.

6. The device (1) according to claim 1, **characterized in that** it comprises remote data transmission means (27), for sending the signal that originates from said optical vision means (4) to a separate display system, which are associated with said grip means (2), a connection cable (28) being provided which is interposed between said data transmission means (27) and said optical vision means (4) and is divided into a first portion (28a), which extends between said data transmission means (27) and said first body (9), and a second portion (28b), which extends between said second body (10) and said optical vision means (4), and connector means (29) being further provided between the first portion and the second portion (28a, 28b) of said connection cable (28) which are interposed between said first body and second body (9, 10).

7. The device (1) according to claim 1, **characterized in that** it comprises lighting means (30) which are associated with the distal end (3a) of said insertion tube (3).

8. The device (1) according to claims 6 and/or 7, **characterized in that** it comprises electric power supply means (31) which are functionally associated with said optical vision means (4) and/or with said lighting means (30) via said connection cable (28) and are associated with said grip means (2).

9. The device (1) according to one or more of claims 6-8, **characterized in that** said connection cable (28) and said connector means (29) are of the USB type.

10. The device (1) according to claim 1, **characterized in that** said insertion tube (3) comprises at least one auxiliary channel (32) which passes inside it and has a first end which is open at the distal end (3a) of said tube and a second end which communicates with an access port (33) which is associated with said second body (10).

11. The device (1) according to one or more of the preceding claims, **characterized in that** said orientation system (5) comprises two of said pairs (11) of flexible actuation elements (12, 13), each one of which comprises a first portion and a second portion (12a, 12b; 13a, 13b) with interposed corresponding temporary connection means (14).

12. The device (1) according to one or more of the preceding claims, **characterized in that** it comprises a protection element (34) which is adapted to protect said first body (9) and the grip means (2) against any forms of contamination.

## Patentansprüche

1. Endoskopievorrichtung (1) mit einem schwenkbaren Kopf, die folgendes umfasst: Greifmittel (2), die mit einem flexiblen Einführschlauch (3) verbunden sind, der am distalen Ende (3a) mit optischen Sichtmitteln (4) versehen ist, wobei ferner ein Orientierungssystem (5), das außerdem für einen Endabschnitt des Einführungsrohrs (3) in der Nähe des distalen Endes (3a) vorgesehen ist, das Verstellmittel (6), die von einem Bediener betätigt werden können und mit den Greifmitteln (2) verbunden sind, und flexible Betätigungsmittel (7) umfasst, die mit den Verstellmitteln (6) zusammenwirken und so angeordnet sind, dass sie durch den Einführschlauch (3) hindurchdringen und mit dem distalen Ende (3a) des Schlauchs verbunden sind, wobei die Endoskopievorrichtung (1) ferner ein Gelenk (8) umfasst, das zwischen dem Greifmittel (2) und dem Einführungsrohr (3) angeordnet ist und einen ersten Körper (9), der stabil mit dem Greifmittel (2) verbunden ist, und einen zweiten Körper (10) umfasst, der stabil mit dem Einführschlauch (3) verbunden ist, die vorübergehend miteinander verbunden werden können, wobei die flexiblen Betätigungsmittel (7) mindestens ein Paar (11) von flexiblen Elementen (12, 13) aufweisen, von denen jedes in einen ersten Abschnitt (12a; 13a) unterteilt ist, der mit den Verstellmitteln (6) verbunden ist und so eingesetzt wird, dass er durch die Greifmittel (2) und den ersten Körper (9) hindurchgeht, und in einen zweiten Abschnitt (12b; 13b), der mit dem distalen Ende (3a) des Einführschlauchs (3) verbunden ist und so eingeführt wird, dass er durch den Schlauch und den zweiten Körper (10) hindurchgeht, und wobei ferner temporäre Verbindungsmittel (14) vorgesehen sind, die zwischen den ersten und den zweiten Abschnitten (12a, 12b; 13a, 13b) eines jeden der flexiblen Betätigungselemente (12; 13) angeordnet sind, wobei die Verbindungsmittel (14) vom magnetischen Typ sind und die Verbindungsmittel (14) für jedes der flexiblen Betätigungselemente (12; 13) des mindestens einen Paares (11) einen ersten Magneten und einen zweiten Magneten (19, 20) umfassen, die mit dem freien Ende des entsprechenden ersten Abschnitts und des zweiten Abschnitts (12a, 12b; 13a, 13b) verbunden sind und sich gegenseitig anziehen können, **dadurch gekennzeichnet, dass** der erste Magnet und der zweite Magnet (19, 20), die mit jedem der flexiblen Betätigungselemente (12; 13) des mindestens einen Paares (11) verbunden sind, eine im Wesentlichen zylindrische Form haben und so angepasst sind, dass sie entlang eines ersten Kanals und eines zweiten Kanals (21, 22) gleiten, die miteinander ausgerichtet sind und in dem ersten Körper beziehungsweise dem zweiten Körper (9, 10) vorgesehen sind.

2. Die Vorrichtung (1) gemäß Anspruch 1, **dadurch gekennzeichnet, dass** die ersten und zweiten Magneten (19, 20) Neodym umfassen.

3. Die Vorrichtung (1) gemäß Anspruch 1, **dadurch gekennzeichnet, dass** sie Verriegelungsmittel (23) zum Verriegeln des Gleitens der ersten Magnete (19) entlang der jeweiligen ersten Kanäle (21) umfassen, die mit dem ersten Körper (9) verbunden sind, um eine Entkopplung durch Zug und/oder Biegung des ersten Magneten und des zweiten Magneten (19, 20) zu ermöglichen, die mit jedem der flexiblen Betätigungselemente (12; 13) des mindestens eines Paares (11) verbunden sind.

4. Die Vorrichtung (1) gemäß Anspruch 1, **dadurch gekennzeichnet, dass** die Verstellmittel (6) mindestens ein Verstellelement (15) umfassen, das auf den Greifmitteln (2) drehbar gelagert ist, und dass die flexiblen Elemente (12, 13) des mindestens einen Paares (11) durch einen Zwischenabschnitt (16) miteinander verbunden sind, der um das Verstellelement (15) gewickelt ist und einen Abschnitt aufweist, der mit diesem integral ist.

5. Die Vorrichtung (1) gemäß Anspruch 1, **dadurch gekennzeichnet, dass** die Verbindungsmittel (14) vom reversiblen, mechanischen Typ sind.

6. Vorrichtung (1) gemäß Anspruch 1, **dadurch gekennzeichnet, dass** sie Datenfernübertragungsmittel (27) umfasst zum Senden des Signals, das von den optischen Sichtmitteln (4) stammt, an ein separates Anzeigesystem, wobei diese mit den Greifmitteln (2) verbunden sind, wobei ein Verbindungskabel (28) vorgesehen ist, das zwischen den Datenübertragungsmitteln (27) und den optischen Sichtmitteln (4) angeordnet ist und in einen ersten Abschnitt (28a), der sich zwischen den Datenübertragungsmitteln (27) und dem ersten Körper (9) erstreckt, und einen zweiten Abschnitt (28b), der sich zwischen dem zweiten Körper (10) und den optischen Sichtmitteln (4) erstreckt, unterteilt ist, und wobei weiter Verbindungsmittel (29) zwischen dem ersten Abschnitt und dem zweiten Abschnitt (28a, 28b) des Verbindungskabels (28) vorgesehen sind, die zwischen dem ersten Körper und dem zweiten Körper (10) angeordnet sind.

7. Die Vorrichtung (1) nach Anspruch 1, **dadurch gekennzeichnet, dass** sie Beleuchtungsmittel (30) umfasst, die mit dem distalen Ende (3a) des Einführschlauchs (3) verbunden sind.

8. Die Vorrichtung (1) nach Anspruch 6 und 7, **dadurch gekennzeichnet, dass** sie elektrische Stromversorgungsmittel (31) umfasst, die mit den optischen Sichtmittel (4) und/oder mit den Beleuchtungsmitteln (30) über das Verbindungskabel (28) funktionell verbunden sind und mit den Greifmitteln (2) verbunden sind.

9. Die Vorrichtung (1) gemäß einem oder mehreren der Ansprüche 6-8, **dadurch gekennzeichnet, dass** das Verbindungskabel (28) und die Anschlussmittel (29) vom USB-Typ sind.

10. Die Vorrichtung (1) gemäß Anspruch 1, **dadurch gekennzeichnet, dass** der Einführschlauch (3) mindestens einen Hilfskanal (32) aufweist, der in seinem Inneren verläuft und ein erstes Ende aufweist, das am distalen Ende (3a) des Schlauches offen ist, und ein zweites Ende, das mit einer Zugangsöffnung (33) in Verbindung steht, die mit dem zweiten Körper (10) verbunden ist.

11. Vorrichtung (1) gemäß einem oder mehreren der vorhergehenden Ansprüche, **dadurch gekennzeichnet, dass** das Orientierungssystem (5) zwei der Paare (11) von flexiblen Betätigungselementen (12, 13) umfasst, von denen jedes einen ersten Abschnitt und einen zweiten Abschnitt (12a, 12b; 13a, 13b) mit dazwischenliegenden entsprechenden temporären Verbindungsmitteln (14) umfasst.

12. Das Gerät (1) gemäß einem oder mehreren der vorhergehenden Ansprüche, **dadurch gekennzeichnet, dass** es ein Schutzelement (34) umfasst, das geeignet ist, den ersten Körper (9) und die Greifmittel (2) vor jeglicher Art von Verschmutzung zu schützen.

## Revendications

1. Dispositif d'endoscopie (1) à tête orientable, comprenant un moyen de préhension (2) qui est associé à un tube d'insertion flexible (3) qui est pourvu à l'extrémité distale (3a) d'un moyen de vision optique (4), un système d'orientation (5) étant en outre prévu pour une partie d'extrémité dudit tube d'insertion (3) à proximité de l'extrémité distale (3a), qui comprend un moyen de réglage (6) qui peut être actionné par un opérateur et qui est associé audit moyen de préhension (2), et un moyen d'actionnement flexible (7) qui coopère avec ledit moyen de réglage (6) et qui est agencé de façon à passer dans ledit tube d'insertion (3) et est associé à l'extrémité distale (3a) dudit tube, le dispositif d'endoscopie (1) comprenant en outre une articulation (8) qui est intercalée entre ledit moyen de préhension (2) et ledit tube d'insertion (3) et qui comprend un premier corps (9) qui est associé de façon stable audit moyen de préhension (2) et un deuxième corps (10) qui est associé de façon stable audit tube d'insertion (3), qui peuvent être associés temporairement l'un à l'autre, ledit moyen d'actionnement flexible (7) comprenant au moins une paire (11) d'éléments flexibles (12, 13), constitués chacun d'une première partie (12a ; 13a), qui est associée audit moyen de réglage (6) et est insérée de manière à passer dans ledit moyen de préhension (2) et ledit premier corps (9), et d'une deuxième partie (12b ; 13b), qui est associée à l'extrémité distale (3a) dudit tube d'insertion (3) et est insérée de manière à passer dans ledit tube et dans ledit deuxième corps (10), et un moyen de connexion temporaire (14) étant en outre prévu, qui est intercalé entre les première et deuxième parties (12a, 12b ; 13a, 13b) de chacun desdits éléments d'actionnement flexibles (12 ; 13), ledit moyen de connexion (14) étant du type magnétique et ledit moyen de connexion (14) comprenant, pour chacun desdits éléments d'actionnement flexibles (12 ; 13) de ladite au moins une paire (11), un premier aimant et un deuxième aimant (19, 20), qui sont associés respectivement à l'extrémité libre de la première partie correspondante et de la deuxième partie (12a, 12b ; 13a, 13b) et qui sont adaptés pour s'attirer mutuellement, **caractérisé en ce que** ledit premier aimant et ledit deuxième aimant (19, 20) associés à chacun desdits éléments d'actionnement flexibles (12 ; 13) de ladite au moins une paire (11) ont une forme substantiellement cylindrique et sont logés de façon à glisser le long d'un premier canal et d'un deuxième canal (21, 22) qui sont mutuellement alignés et qui sont placés, respectivement, dans ledit premier corps et ledit deuxième corps (9, 10).

2. Dispositif (1) selon la revendication 1, **caractérisé en ce que** lesdits premier et deuxième aimants (19, 20) comprennent du néodyme.

3. Dispositif (1) selon la revendication 1, **caractérisé en ce qu'**il comprend un moyen de blocage (23) pour bloquer le glissement desdits premiers aimants (19) le long des premiers canaux respectifs (21), qui est associé audit premier corps (9) afin de permettre le désaccouplement par traction et/ou flexion du premier aimant et du deuxième aimant (19, 20) qui sont associés à chacun desdits éléments d'actionnement flexibles (12 ; 13) de ladite au moins une paire (11).

4. Dispositif (1) selon la revendication 1, **caractérisé en ce que** ledit moyen de réglage (6) comprend au moins un élément de réglage (15) qui est supporté de manière à pouvoir être mis en rotation par ledit moyen de préhension (2) et **en ce que** les éléments flexibles (12, 13) de ladite au moins une paire (11) sont mutuellement reliés par une partie intermédiaire (16) qui est enroulée autour dudit élément de réglage (15) et qui a une partie qui est intégrée à celui-ci.

5. Dispositif (1) selon la revendication 1, **caractérisé en ce que** ledit moyen de connexion (14) est du type mécanique réversible.

6. Dispositif (1) selon la revendication 1, **caractérisé en ce qu'**il comprend un moyen de transmission de données à distance (27), pour envoyer le signal qui provient dudit moyen de vision optique (4) à un système d'affichage distinct, qui est associé audit moyen de préhension (2), un câble de connexion (28) étant prévu, qui est intercalé entre ledit moyen de transmission de données (27) et ledit moyen de vision optique (4) et est divisé en une première partie (28a), qui s'étend entre ledit moyen de transmission de données (27) et ledit premier corps (9), et une deuxième partie (28b), qui s'étend entre ledit deuxième corps (10) et ledit moyen de vision optique (4), et un moyen formant connecteur (29) étant en outre placé entre la première partie et la deuxième partie (28a, 28b) dudit câble de connexion (28) qui sont intercalées entre lesdits premier corps et deuxième corps (9, 10).

7. Dispositif (1) selon la revendication 1, **caractérisé en ce qu'**il comprend un moyen d'éclairage (30) qui est associé à l'extrémité distale (3a) dudit tube d'insertion (3).

8. Dispositif (1) selon les revendications 6 et/ou 7, **caractérisé en ce qu'**il comprend un moyen d'alimentation électrique (31) qui est associé fonctionnellement audit moyen de vision optique (4) et/ou audit moyen d'éclairage (30) par l'intermédiaire dudit câble de connexion (28) et qui est associé audit moyen de préhension (2).

9. Dispositif (1) selon l'une ou plusieurs des revendications 6 à 8, **caractérisé en ce que** ledit câble de connexion (28) et ledit moyen formant connecteur (29) sont du type USB.

10. Dispositif (1) selon la revendication 1, **caractérisé en ce que** ledit tube d'insertion (3) comprend au moins un canal auxiliaire (32) qui passe dans celui-ci et qui a une première extrémité qui est ouverte à l'extrémité distale (3a) dudit tube et une deuxième extrémité qui communique avec un orifice d'accès (33) qui est associé audit deuxième corps (10).

11. Dispositif (1) selon l'une ou plusieurs des revendications précédentes, **caractérisé en ce que** ledit système d'orientation (5) comprend deux desdites paires (11) d'éléments d'actionnement flexibles (12, 13), constitués chacun d'une première partie et d'une deuxième partie (12a, 12b ; 13a, 13b) avec un moyen de connexion temporaire correspondant intercalé (14).

12. Dispositif (1) selon l'une ou plusieurs des revendications précédentes, **caractérisé en ce qu'**il comprend un élément de protection (34) qui est adapté pour protéger ledit premier corps (9) et le moyen de préhension (2) contre toute forme de contamination.
